# EUROPEAN PATENT SPECIFICATION

(11) **EP 3 072 389 B1**
(45) Date of publication and mention of the grant of the patent: **22.09.2021**
(21) Application number: 14858723.1
(22) Date of filing: 30.10.2014
(51) Int. Cl.: A01H 4/00, C12M 3/00, A01G 7/06

(54) **METHOD FOR THE IN VITRO MICROPROPAGATION OF PLANT MATERIAL AND METHOD FOR LARGE-SCALE AND LARGE-VOLUME PRODUCTION OF CLONED PLANT SEEDLINGS READY FOR FIELD GROWTH**
VERFAHREN ZUR IN-VITRO-MIKROVERMEHRUNG VON PFLANZENMATERIAL UND VERFAHREN ZUR GROSSFLÄCHIGEN UND GROSSVOLUMIGEN HERSTELLUNG VON GEKLONTEN PFLANZENSETZLINGEN FÜR FELDWACHSTUM
PROCÉDÉ DE MICROPROPAGATION IN VITRO DE MATÉRIEL DE PLANTE ET PROCÉDÉ DE PRODUCTION, À LARGE ÉCHELLE ET EN VOLUME ÉLEVÉ, DE SEMIS CLONAUX DE PLANTES PRÊTES POUR UNE CROISSANCE EN CHAMP

(30) Priority: 30.10.2013 US 201361897680 P
(43) Date of publication of application: 28.09.2016
(73) Proprietor: Suzano S.A., Pituba, City of Salvador,State of Bahia 41810-012 (BR)
(72) Inventor: PENCHEL, Ricardo Miguel, 08900-000 São Paulo - SP (BR); REIS, Jocemar Palauro dos, 29192-204 Aracruz - ES (BR); OLIVEIRA, Mila Liparize de, 12301-572 Villa Branca, Jacarei - SP (BR)
(74) Representative: Herzog IP Patentanwalts GmbH
(86) International application number: PCT/BR2014/000391
(87) International publication number: WO 2015/061871

(56) References cited:
- MCALISTER B ET AL: "Use of the temporary immersion bioreactor system (RITA(R)) for production of commercial Eucalyptus clones in Mondi Forests (SA)", PLANT CELL, TISSUE AND ORGAN CULTURE, KLUWER ACADEMIC PUBLISHERS, DO, vol. 81, no. 3, 1 June 2005 (2005-06-01), pages 347-358, XP019268580, ISSN: 1573-5044
- 309 ET AL: "Scientia ForeStaliS A", , 1 January 2011 (2011-01-01), pages 309-315, XP55343713, Retrieved from the Internet: URL:http://www.ipef.br/publicacoes/scienti a/nr91/cap03.pdf
- Micropropagação De ET AL: "MILA LIPARIZE DE OLIVEIRA", , 1 January 2009 (2009-01-01), XP55344135, Retrieved from the Internet: URL:http://locus.ufv.br/bitstream/handle/1 23456789/3025/texto completo.pdf?sequence=1&isAllowed=y [retrieved on 2017-02-09]
- RAZZOLI ET AL: "Conditioning properties of social subordination in rats: Behavioral and biochemical correlates of anxiety", HORMONES AND BEHAVIOR, ACADEMIC PRESS, NEW YORK, NY, US, vol. 50, no. 2, 1 August 2006 (2006-08-01) , pages 245-251, XP005533860, ISSN: 0018-506X, DOI: 10.1016/J.YHBEH.2006.03.007
- NICOLAS NIEMENAK ET AL: "Regeneration of somatic embryos in Theobroma cacao L. in temporary immersion bioreactor and analyses of free amino acids in different tissues", PLANT CELL REPORTS, SPRINGER, BERLIN, DE, vol. 27, no. 4, 10 January 2008 (2008-01-10), pages 667-676, XP019587777, ISSN: 1432-203X
- WATT M. PAULA: "The status of temporary immersion system (TIS) technology for plant micropropagation", AFRICAN JOURNAL OF BIOTECHNOLOGY, vol. 11, no. 76, 20 September 2012 (2012-09-20), XP55338606, DOI: 10.5897/AJB12.1693
- LI-HUA ZHU ET AL: "Optimisation of growing conditions for the apple rootstock M26 grown in RITA containers using temporary immersion principle", PLANT CELL, TISSUE AND ORGAN CULTURE, KLUWER ACADEMIC PUBLISHERS, DO, vol. 81, no. 3, 1 June 2005 (2005-06-01), pages 313-318, XP019268581, ISSN: 1573-5044
- CASTRO ET AL.: 'Eucalyptus (Eucalyptus grandis Hill ex Maiden) micropropagation in a temporary immersion system.' AGRICULTURA TÉCNICA vol. 2, no. 1, 2002, pages 68 - 78, XP008183614
- GONZALEZ ET AL.: 'Multiplicación in vitro de Eucalyptus globulus mediante sistema de inmersión temporal.' BOSQUE vol. 32, no. 2, 2011, pages 147 - 154, XP055338601
- WATT.: 'The status of temporary immersion system (TIS) technology for plant micropropagation.' AFRICAN JOURNAL OF BIOTECHNOLOGY vol. 11, no. 76, 2012, pages 14025 - 14035, XP055338606
- CORREIA.: 'Micropropagação em biorreatores de imersao temporária e enraizamento de miniestacas e microestacas de clones hibridos de Eucalyptus globulus.' DISSERTAÇÃO DE MAGISTER SCIENTIA APRESENTADA À UNIVERSIDADE FEDERAL DE VIÇOSA 2011, XP055338607
- Ricardo Gonz?lez ET AL: "Multiplicaci?n in vitro de Eucalyptus globulus mediante sistema de inmersi?n temporal In vitro multiplication of Eucalyptus globulus by temporary immersion system", BOSQUE, 1 January 2011 (2011-01-01), pages 147-154, XP055338601, Retrieved from the Internet: URL:http://www.scielo.cl/pdf/bosque/v32n2/ art05.pdf [retrieved on 2017-01-25]

## Description

### FIELD OF THE INVENTION

The present invention relates to a process for the production of clonal plants of arboreal species, especially eucalyptus, in large-scale and high-volume, whereby the step of vegetative propagation, performed in vitro, occurs under conditions such that the obtaining of healthy and vigorous microcuttings of the arboreal species is maximized, while the rate of hyperhydricity of the micropropagation phase in the temporary immersion regime is minimized.

### FUNDAMENTALS OF THE INVENTION

Micropropagation consists of a technique of plant tissue culture which is used as a basis for the formation of seedlings, being a practical, safe and fast method of mass propagation of plants in a reduced space. Conventionally, micropropagation is performed via the semi-solid system, typically in a gelling medium with agar. However, this is still a costly practice, allowing room for the use of equipment and new technologies with equipment called bioreactors that help in the production of plants in large scale operation and reduce the high cost of labor.

Bioreactors are devices for the cultivation of seedlings in a regime of temporary or permanent immersion to obtain plants. The bioreactor consists of a set of containers with compartments and accessories for optimal management of key growth factors to maximize the biological reactions. By means of this equipment, it is possible to obtain higher rates of multiplication of the culture by the use of a liquid nutrient medium, injection and exchange of gases, e.g. air enriched with carbon dioxide, inside the container, as well as better physiological control of cultivation conditions and process automation. There are various types of bioreactors, which may vary depending on the culture container and culture medium, the immersion-type (temporary or permanent), and their functionality. The temporary immersion systems best known in Brazil are RITA® (temporary automated immersion container - for example, created by CIRAD-France) and BIT® (temporary immersion bioreactor - for example, created by Embrapa-Cenargen) .

Due to the potential of the micropropagation technique, there is growing interest in this technology as demonstrated by the prior art. For example, document WO 2012/061950 describes a method and bioreactor for the micropropagation of an Antarctic plant species, wherein the method is characterized by the use of a nutrient medium and appropriate conditions for the development of said plant species, and additionally employs a protocol for supplying UV radiation and temperature control for the production of valuable metabolites for medical and cosmetic use.

Document WO 2012/156440 describes a temporary immersion system and method of micropropagation of plant species characterized by the provision of appropriate conditions for the development of propagules, such as lighting, a temperature and immersion system with intervals ranging from 1 to 10 times per day, and immersion time of 1 to 2 minutes or 3 to 4 minutes depending upon the plant species being developed.

Watt (see Watt, MP "The status of temporary immersion system (TIS) technology for plant micropropagation." African Journal of Biotechnology, Vol. 11 (76), p. 14025-14035, 09/20/2012) provided a retrospective of the micropropagation of plants, particularly with respect to proposals to reduce hyperhydricity, citing as the main factors, the shortening of the immersion time and increasing periods of rest (time between two successive immersions).

Gonzalez (see Gonzalez, R. et al. "Multiplication in vitro of Eucalyptus globulus by temporal immersion system." Bosque 32(2) : 147-154, 2011) reported the factors affecting the stage of in vitro multiplication of *Eucalyptus globulus* by temporary immersion and concludes that the rate of multiplication of microcuttings of this arboreal species increases significantly when a soak time of 1-2 min and a frequency of immersion (rest period) for 12 hours are employed. These authors also concluded that hyperhydricity increases with the concentration of nutrients and sucrose.

Oliveira (see Oliveira ML, "Micropropagation of clones of Eucalyptus grandis x E. urophylla in a temporary immersion bioreactor." Master's Thesis Dissertation. Federal University of Viçosa. 2011) reports the study of in vitro micropropagation of two eucalyptus species, concluding that there is a high percentage of occurrence of hyperhydricity in the development of propagules when the technique of temporary immersion is employed. The author also concluded that "there is a need for adjustment in the crop management phases of multiplication and elongation to obtain shoots with greater vigor, able to root in ex vitro conditions in order to make this technique feasible on a large scale."

The aforementioned prior art shows that although there has been great progress in the technique of in vitro micropropagation of plants, the problem of hyperhydricity remains to be solved and, at the same time, employing suitable conditions to obtain clonal seedlings of plants, particularly of the arboreal species, and more particularly of the eucalyptus species, enabling the production of said cloned seedlings on a large scale and high volume and to achieve commercial production of healthy and luxuriant plants.

### SUMMARY OF THE INVENTION

The present invention refers to the obtaining of clonal plant seedlings, in particular of arboreal species, particularly the species of eucalyptus, using the technique of in vitro micropropagation of plant material, comprising the steps of multiplication, elongation, rooting, acclimatization, and growth and acclimatization of seedlings ready for commercialization.

In a first embodiment, the present invention relates to a process for in vitro micropropagation of plant material, especially of arboreal species, particularly from a species of eucalyptus, comprising the steps of multiplication and elongation of shoots developed from plant propagules by the temporary immersion method, as follows: (a) in the step of multiplication of the shoots, the material to be propagated is placed on a suitable support in a first container (plant propagation container) and is subjected to management of temporary immersion in a liquid nutrient medium from a second container (storage container of a liquid medium), said management comprising alternated periods of immersion and rest, with the immersion time being preferably at least 1 minute, preferably at most for 3 minutes, more preferably for 1 to 2 minutes, with the resting period preferably at least 2 hours, preferably for up to 4 hours, more preferably for 2 to 3 hours; (b) in the step of elongation of shoots, the propagation material is subjected to management of temporary immersion, preferably employing the same nutrient medium of step (a), said management comprising alternated periods of immersion and rest, the immersion time being preferably for at least 20 seconds, preferably for at most 60 seconds, more preferably for 20 to 30 seconds, with the rest period preferably being at least 4 hours, preferably for at most 6 hours, more preferably for 4 to 5 hours; said method being characterized by the fact that, in said elongation step, said immersion periods are significantly shorter and said rest periods are longer compared with the periods of immersion and rest, respectively, in the multiplication step.

In a second embodiment, the invention relates to a process for the production of clonal plant seedlings ready for field development comprising the steps of: (i) subjecting the plant propagules to vegetative propagation conditions of temporary immersion in vitro comprising the stages of: (a) multiplication of the shoots in which the material to be spread on suitable support is placed in a first container (micropropagation container) and said material is subjected to the management of temporary immersion in a liquid nutrient medium, from a second container (liquid medium storage container), with said management comprising alternating periods of immersion and rest, with the immersion time preferably at least 1 minute, preferably at for most 3 minutes, more preferably for 1 to 2 minutes, and with the period of rest being preferably at least 2 hours, preferably for at most 4 hours, more preferably for 2 to 3 hours; (b) elongation of shoots in which the material is subjected to propagation management of temporary immersion, preferably using the same nutrient medium of stage (a), said management comprising alternating periods of rest and immersion, with the immersion period being preferably at least 20 seconds, preferably for at most 60 seconds, more preferably for 20 to 30 seconds, and the rest period being preferably at least 4 hours, preferably for at most 6 hours, more preferably for 4 to 5 hours; (ii) rooting and acclimatization of the multi buds obtained in step (i) in containers and conditions suitable to maintain plant turgor and reduce evaporative demand; and (iii) growth and acclimatization of the microcuttings obtained in step (ii) .

### BRIEF DESCRIPTION OF THE DRAWINGS

Figure 1 illustrates the process of the present invention showing the micropropagated / microcutting of the material at each step.

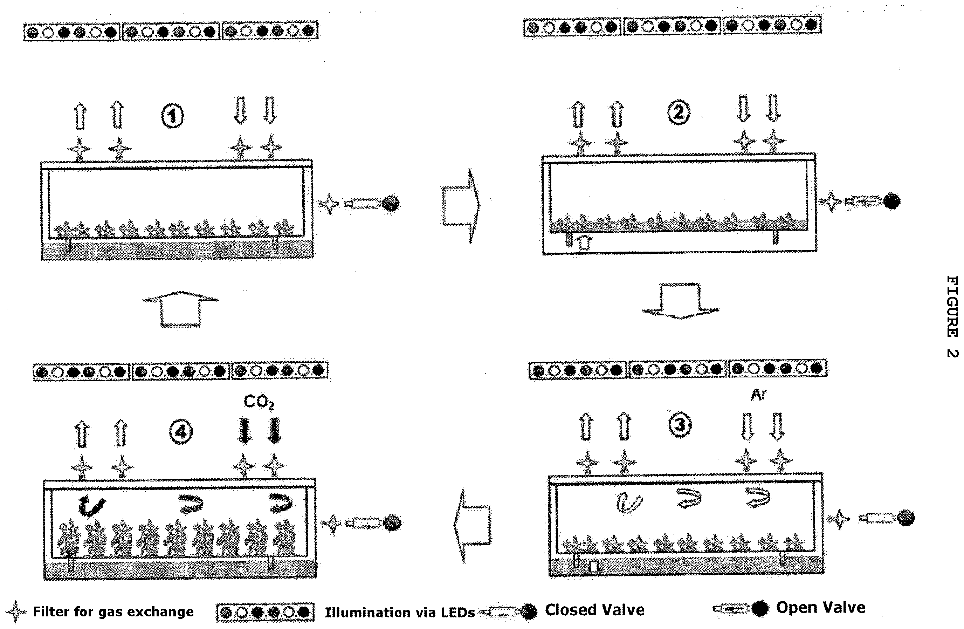

Figure 2 shows the step of in vitro micropropagation in a regime of temporary immersion in a liquid nutrient medium.

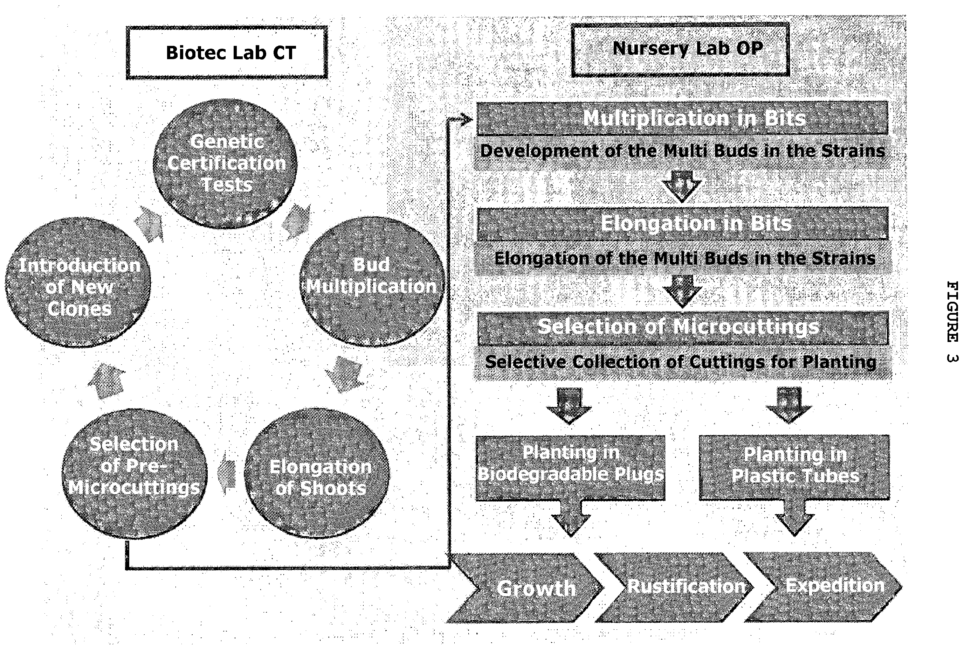

Figure 3 shows the development stages of clonal seedlings in large scale and high volume, from selection of the correct clone to market introduction.

### DETAILED DESCRIPTION OF THE INVENTION

The production system of microcuttings of clonal arboreal species, such as eucalyptus, in high scale and high productivity in temporary immersion bioreactors has as its main objective the gradual replacement of traditional clonal gardens of operational forest nurseries by plant biofactories, aimed at producing industrial micropiles for subsequent rooting and acclimatization in specific greenhouses (cloches).

In bioreactors, it is possible to obtain higher rates of multiplication and crop growth by continuous use of a liquid nutrient medium, supplemented with air injection and carbon dioxide enrichment and photosynthetically active radiation with light-emitting diode lamps inside the plant containers. Thus, the management of production of shoots and phytomass is best optimized via automated manipulation of key growth factors, enabling better physiological control of culture conditions.

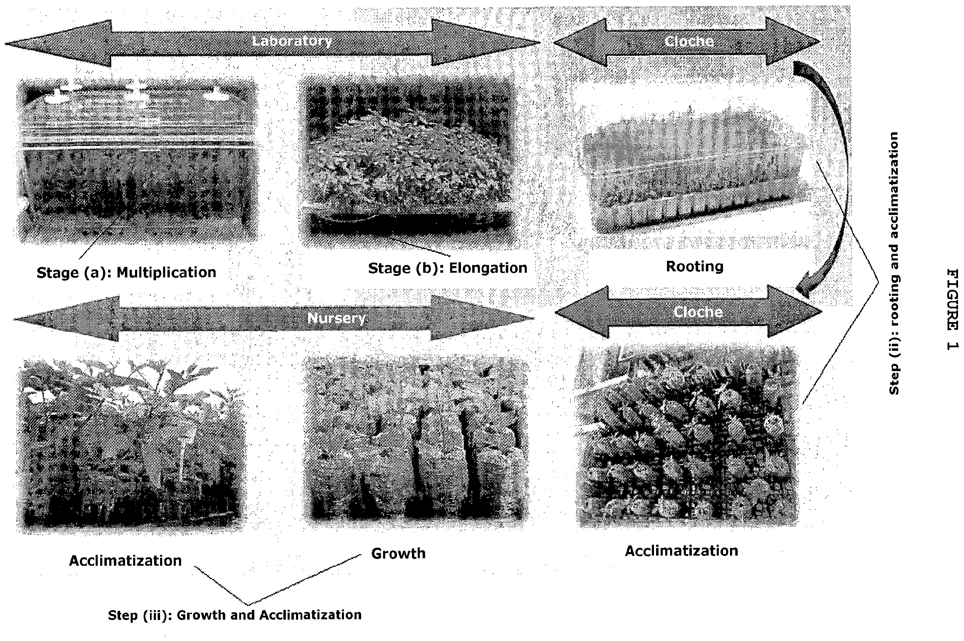

What has been sought in this technique is the minimization of hyperhydricity, which causes disturbances and degeneration of the cloned plantlets obtained by the method of temporary immersion and, at the same time, retains the advantages of this technique in terms of production costs and health/vigor of the produced seedlings. This challenge was met by the inventors of this invention who have created a method in which alternating periods of immersion and rest of the material in vegetative propagation are differentiated in the stages of multiplication and elongation of shoots. This procedure has made it possible to reduce hyperhydricity and maximize the production of clonal plantlets, in particular arboreal species, and most preferably of the eucalyptus species. In Figure 1, these two stages are represented as activities in the laboratory, wherein micropropagation, i.e. the step of including the stages of
multiplication and elongation, generally takes a period of about 45 days.

The process of the present invention has been customized to meet the physiological demands for the vegetative propagation of arboreal species, especially eucalyptus, allowing the intensive and precision development of plants, differentiated to result in the best quality standard and uniformity and vigor of shoots produced, providing greater rooting of microcuttings and subsequent better acclimatization and early yield of clonal plantlets for industrial use on a large-scale.

The process of the present invention allows the obtaining of productivity at least 30 times greater than the traditional method of producing plantlets from commercial nurseries, in addition to providing a 33% reduction in cycle time for production of a clonal plantlet. Additionally, the operational deployment of the process of the invention in nurseries provides a reduction of manpower and a reduction in the construction cost of clonal gardens.

The process of the invention solves, among others, the following problems:
- Production of clonal plantlets on a large scale and high volume, with uniformity in quality, for maximum efficiency and productivity of vegetative propagation of arboreal plants, for example, eucalyptus, with reduced operating costs and production cycles.
- Reduction of physiological abnormalities and disorders related to hyperhydricity of the explants, a phenomenon commonly referred to as plant vitrification. The management of gas exchange is one of the advantages of the invention, which is preferably performed in a bioreactor of the BIT type, but may be performed in a bioreactor of another type, such as RITA, which practically eliminates these biological problems.
- Reduction of the cost of production of eucalyptus clonal seedlings associated with reduced labor involved in clonal gardens in the operational process.
- Uniformity of the pattern of physiological quality of plant matrices and microcuttings in vegetative propagation, performed in a temporary immersion bioreactor, in contrast with the lack of uniformity of plants and cuttings obtained from plants grown in clonal gardens, where environmental conditions are not uniform.
- Difficulties of the vegetative propagation of species and clones of arboreal species, including eucalyptus, recalcitrants are contoured with vegetative propagation in vitro in bioreactors, allowing the use of new and more productive clones that would not be produced by conventional means.
- Clonal cleaning, by obtaining mother-plants and crops free of pathogenic microorganisms and, also, cultures of high genetic fidelity of single clone without mixing with other clones.
- Acceleration of breeding programs by multiplication of superior clones, aiming to produce clonal plantlets in less time and in greater quantity and reduced space, in addition to the rejuvenation of selected clones and matrices.

It is important to note that the use of microshoots (or products of micropropagation) obtained according to the process of the invention, for direct planting in specific containers (which can be tubes, plugs, styrofoam trays or otherwise), replaces the supply of shoots to the market from known clonal gardens (either clonal mini-gardens or clonal micro-gardens). This is a great advantage of the technique employed in the micropropagation process of the present invention, and one that, with the production of eucalyptus, presents a return in terms of unmatched quantity and quality which is not comparable with the technique used in clonal gardens.

### Description of the Nutritive Media Culture in the Bioreactor According to the Invention

Throughout the process of growth and development of plants in the bioreactor, two basic culture media can be used, as shown below. These media are chemically modified according to the present invention to attend specific clones, according to the need and physiological stage of the culture. The media are supplemented with growth phytoregulators, fostering bud multiplication, elongation of shoots and pre-induction of ex-vitro rooting of microcuttings.

The types of concentrations of growth regulators, as well as their use in the management of the bioreactor process are those commonly employed in the art, with proper adaptation to the cultivation of the desired species for propagation, preferably including arboreal species, and more preferably eucalyptus.

**Table 1: Liquid nutrient medium type JADS (see Correia, D. 1993. "Growth and development of buds in the multiplication of eucalyptus in vitro in liquid and solid culture medium." Master's Thesis Dissertation in Forestry Science, Luiz de Queiroz College of Agriculture, Piracicaba)**

| **JADS** | | | |
|---|---|---|---|
| **Type** | **Reagent** | **Formulation** | **Concentration (mg/L)** |
| **Inorganics** | Amonium Nitrate | NH₄NO₃ | 1650 |
| | Potassium Nitrate | KNO₃ | 809 |
| | Monobasic potassium phosphate | KH₂PO₄ | 408 |
| | Magnesium sulfate heptahydrate | MgSO₄.7H₂O | 739.6 |
| | Calcium nitrate tetrahydrate | Ca(NO₃)₂.4H₂O | 1181 |
| | Iron sulfate heptahydrate | FeSO₄.7H₂O | 55.6 |
| | Disodium EDTA | NA₂EDTA | 74.6 |
| | Manganese sulfate heptahydrate | MnSO₄.H₂O | 17 |
| | Zinc sulfate heptahydrate | ZnSO₄.7H₂O | 4.32 |
| | Molybdate dihydrate | Na₂MoO₄.2H₂O | 0.15 |
| | Cupric sulfate pentahydrate | CuSO₄.5H₂O | 1.25 |
| | Cobalt chloride hexahydrate | CoCl₂.6H₂O | 0.25 |
| | Boric Acid | H₃BO₃ | 3.1 |
| **Organics** | Nicotinic Acid | | 0.5 |
| | Pyridoxine | | 0.5 |
| | Thiamine | | 5 |
| | Meso Inositol | | 100 |
| | Glutamine | | 146 |
| | Arginine | | 7 |
| | Cysteine | | 5 |
| | Calcium pantothenate | | 2.4 |

**Table 2: Liquid nutrient medium MS type (see Murashige, T. & Skoog, F. 1962. A revised medium for rapid growth and bioassay with tobacco tissue cultures. Physiologia Plantarum 15:473-497**

| **MS** | | | |
|---|---|---|---|
| **Type** | **Reagent** | **Formulation** | **Concentration (mg/L)** |
| **Inorganics** | Amonium Nitrate | NH₄NO₃ | 1650 |
| | Potassium Nitrate | KNO₃ | 1900 |
| | Monobasic potassium phosphate | KH₂PO₄ | 170 |
| | Boric Acid | H₃BO₃ | 6.2 |
| | Molybdate dihydrate | Na₂MoO₄ 2H₂O | 0.25 |
| | Calcium chloride dihydrate | CaCl₂.2H₂O | 440 |
| | Cobalt chloride hexahydrate | CoCl₂.6H₂O | 0.025 |
| | Magnesium sulfate heptahydrate | MgSO₄.7H₂O | 370 |
| | Manganese sulfate heptahydrate | MnSO₄.H₂O | 16.9 |
| | Zinc sulfate heptahydrate | ZnSO₄.7H₂O | 8.6 |
| | Cupric sulfate pentahydrate | CuSO₄.5H₂O | 0.025 |
| | Iron sulfate heptahydrate | FeSO₄.7H₂O | 27.85 |
| | Disodium EDTA | Na₂EDTA.2H₂O | 37.3 |
| **Organics** | Myo Inositol | | 100 |
| | Thiamine HCl | | 10 |
| | Nicotinic acid | | 0.5 |
| | Piridoxine HCl | | 0.5 |
| | L-Glutamine | | 100 |

### Planting of Microcuttings in Biodegradable Plugs

As shown in Figure 1, the elongated shoots are transferred from the laboratory to a controlled environment, known by the denomination of mini-greenhouse, where the stages of Rooting and Acclimatization are performed. To do so, the shoots are separated and the subjects are each placed in a container containing a solid medium, with such containers being placed in trays or on shelves. There are several specific types of containers for the rooting of clonal seedlings such as the types cited below.

### Planting containers and trays:

Ellepot EP: Degradable plastic tubete produced by the Danish company Ellegaard®, comprising: white polyester or cellulose membrane and holes with cylindrical shape; Capacities: 85 cc and 110 cc; Dimensions: 30 x 100 mm and 40 x 100 mm.

Jiffy Pellet: Biodegradable plastic tubete produced by the Norwegian company Jiffy®, comprising expandable insert with coconut fiber and sphagnum peat, cylindrical; Capacities 85 cc and 100 cc; Dimensions: 30 x 100 mm and 36 x 100 mm. Care-Free® type Peat Pellet Expandable Wafers: Dimensions: 30x100 and 36x100, a combination of dried and pressed sphagnum peat, perlite and mineral additives, coated with a permeable membrane and biodegradable PLA, produced by the Canadian branch of the Norwegian company Jiffy®.

Air-Tray® tray 117: Intended to serve as a support for the CareFree® Jiffy-Pellets 30x100 mm, with 390 x 590 mm (2,301 cm2), with 117 cells, and with an average area of 19.6 cm2/cell, for a tray with 100% occupancy (treatment T-1) .

Air-Tray® tray 64: Intended to serve as a support for the CareFree® Jiffy-Pellets 36x100 mm, with 380 x 380 mm (1,444 cm2), with 64 cells, with an average area of 22.5 cm 2/cell for a tray with 100% occupancy (treatment T-2).

After proper allocation of the pellets in the trays, they must be placed with their bottom (base) in contact with a shallow layer of pure water so that the pellets in the trays are not covered (or float) in the water. This is the process of expansion of the pellets, which in this case "rehydrates" them by capillarity. It is important to note, that the base of the pellets should be kept in contact with this layer of water until fully expanded. If the water depth is reduced (due to absorption by the pellets), the water must be replaced. There must be no concern about "excess water." This operation takes only a few minutes. If one wishes to speed up the expansion process, one can make use of heated water of approximately 45°C. In this case, it is essential to carry out the expansion of the pellets some minutes in advance, such that their temperature approaches room temperature, before being taken to receive the microcuttings.

### Acclimatization Technique of Micropropagated Eucalyptus Seedlings

In general, in a population of micropropagated eucalyptus plants, it is likely that a small subpopulation will present anatomical, morphological and physiological abnormalities due to imbalances of the conditions inside the bioreactor and other flasks in the micropropagation. All these changes are associated with unsuitable conditions for the process of rooting microcuttings and subsequent acclimatization of seedlings, and thus reduce survival rates and the quality of seedlings.

The application of acclimatization techniques aims to provide greater graduation in the transition between the in vitro (bioreactor) and the external (nursery) environment. Among these techniques, we highlight the use of *mist and fog* systems, which help to maintain the plant turgor and decrease evaporative demand. Shading helps to reduce high levels of brightness from the external environment and also helps to reduce the temperature and stress of the plants in acclimation.

Alternatively, other products and protected environments are used in the planting of microcuttings produced in bioreactors. Products used include substrates stabilized by biodegradable polymers for planting in wood fiber containers, and coconut fiber and *Sphagnum* peat. These containers function as pressed pellets and are expandable when hydrated.

### Cultivation system with Fog and Cloche

This system generates a mist of extremely small water droplets, with diameters smaller than 15 micrometers, which are nebulized within the cloche environment using specific nozzles or an ultrasonic nebulizer, generating an ultrathin (< 5 µm) dry fog. These droplets are so small that they remain suspended in the air until evaporated, contributing to the increase in humidity and temperature reduction and, at the same time, avoiding leaf wetness. These water droplets in suspension are able to maintain a very thin film of water vapor on both leaf surfaces, which keeps the seedlings turgid and temperature controlled.

The fog system is used in conjunction with the cloche structure, preferably a structure designed specifically for rooting and acclimatization of eucalyptus. The mini-greenhouse consists of the assembly of a mini-greenhouse or over-tunnels within a normal greenhouse, kept suspended on rounded tables. During the growth and development of plantlets in the mini-greenhouse, a sub-surface irrigation system (*floating*) is utilized, which provides temporary immersions of the base of the biodegradable plugs in water or nutrient solution which is used to supply nutrients to the plants without wetting the aerial part of the seedlings.

### Microcuttings Transplant Technique in Biodegradable Plugs

The planting system of the microcuttings produced in the bioreactor in biodegradable containers consists of selecting standard microcuttings of satisfactory quality and inserting the base of the microcuttings into bags or previously hydrated pressed pellets (plugs), as described above.
• Jiffy Rehydration and Expansion Stage:
- After placing the pellets in the trays, they must be positioned with their bottom (base) in contact with a shallow layer of pure water at room temperature so that the pellets in the trays are not covered (or float) in the water, until full expansion, before planting the microcuttings.
- Rehydration of the pellets is accomplished only by capillarity and not by their submersion in water. It is important to note that the base of the pellets should be kept in contact with the layer of water until they are fully expanded. If the water level is reduced (due to absorption by the pellets), it must be replaced. There must be no concern with regard to excess water. This operation takes only a few minutes.
- If one wishes to speed up the expansion process, one can make use of water heated to about 45°C. In this case, it is essential to perform the pellet expansion a few minutes early, so that their temperature approaches room temperature before being taken to receive the microcuttings during planting.
   By way of non-limiting example, some information is provided about the techniques available to perform the planting of the micropiles produced in the bioreactor.

• Technical Specifications of Plugs and Trays (PLA Net = biodegradable corn starch resin)

| Product | Objective |
|---|---|
| Jiffy-7 Care Free Horticulture Peat Plug, 30 x 100 mm, 6 mm indent, PLA net | Planting of microcuttings to produce seedlings for field planting |
| Jiffy-7 Care Free Horticulture Peat Plug, 30 x 80 mm, 6 mm indent, PLA net | Planting of microcuttings to produce seedlings for field planting |
| Jiffy-7 Forestry Peat Pellet, 18 x 25 mm, 9 mm indent, PLA net | Direct planting of the microcutting in mini-plug to produce seedlings for planting in clonal garden |
| Jiffy-7 Forestry Peat Pellet, 18 x 32 mm, 9 mm indent, PLA net | Direct planting of the microcutting in mini-plug to produce plantlets for planting in clonal garden |
| Jiffy-7 Horticulture Peat Pellet, 36 x 75 mm, TP 40 mm, PLA net | Transplanting of rooted microshoot in the 18x25 mini-plug to the 36x75 plug and field planting |
| Jiffy-7 Horticulture Peat Pellet, 36 x 75 mm, TP 25 mm, PLA net | Transplanting of rooted microshoot in the 18x25 mini-plug to the 36x75 plug and field planting |
| New Mini-18 mm thermo form tray para J7 pellet 18x32; tray size 33 x 45 x 4 cm | Trays for supporting the mini-plugs and plantlet growth. |

• Spacing Management of Seedling Trays:

| **Type of Tubete** | **Production Phase** | **Duration (days)** | **Density (cm² / shoot)** | **Capacity (qty / tray)** | **Capacity (% / tray)** |
|---|---|---|---|---|---|
| | | | | | |
| Jiffy 30x100mm | Rooting | 10 | 20 | 117 | 100 |
| | Acclimatization | 10 | 39 | 59 | 50 |
| | Growth | 15 | 96 | 24 | 20 |
| | Harding | 15 | 96 | 24 | 20 |
| | | | | | |
| Jiffy 36x100mm | Rooting | 10 | 23 | 64 | 100 |
| | Acclimatization | 10 | 45 | 32 | 50 |
| | Growth | 15 | 90 | 16 | 20 |
| | Hardening | 15 | 90 | 16 | 20 |

• Irrigation management:
- The application of the depth of irrigation water should be adjusted to achieve the field capacity of each substrate, especially at the stage of rooting (CV) and Acclimation (CA), where the Ellepot and Jiffy must not be saturated with water.
- In the greenhouse, irrigation management should produce an irrigation interval of short duration and intervals between irrigations, for example, 6 seconds duration every 10-15 minutes between irrigations, to increase the level of relative humidity without irrigation of the substrate occurring.
- In acclimatization, irrigation management should allow complete wetting of the substrate of the Ellepot and Jiffy, without causing excess and saturation of the same. The shading, for example, of the shading screen type, must remain extended only during the hottest hours of the day, for example, from 11am to 2pm.
- To the contrary, in the Stages of Growth (PC) and Rustification (PR), the Ellepot and Jiffy should be almost saturated, due to the need for the greater depth of water to compensate for evaporation losses from the container, root uptake and leaf transpiration.
- Additionally, in the growth and rustification terrace, irrigation of the Ellepot and Jiffy is recommended with an irrigation rod or shower head for uniform wetting of the entire substrate.

• Monitoring of Substrate Humidity:
- During the process of rooting and seedling growth in the Ellepot and Jiffy container, it is necessary to monitor the moisture content of the substrate in order to recommend the irrigation shift (duration and interval) so that substrate moisture remains within the ideal range of field capacity.
- These instant, real-time measurements of substrate moisture must be performed using a portable moisture meter + conductivity + temperature, for example, the Delta-T brand, model WET Sensor. This equipment must be calibrated in the laboratory for the substrate being used in the test.
- The humidity sensor must be inserted in at least two positions (depths) within the Ellepot and Jiffy, because only the tip of the sensor is able to accurately record the values in the rhizosphere region. In the CV, this position should be shallow (2-3 cm), in the CA this position should be median (3-6 cm) and in the PC position it should be at the bottom (7-9 cm).
- Between one measurement and the other it is necessary to wait for a period of time (10-15 seconds) for sensor stabilization in the moist substrate. In the same Ellepot or Jiffy, the 1st measurement must be processed at a point of lower humidity, and the 2nd measurement at a point of higher humidity.
- We recommend the following ranges of moisture for the substrate used in the test, measured with the wet sensor, as shown below:

| **Substrate Humidity** | **Humidity Mecprec (%, v/v)** | **Humidity Ellepot and Jiffy (%, v/v)** | **Observation** |
|---|---|---|---|
| Saturated | >40% | **< 50 %** | **High** excess water |
| Semi-saturated | 21 to 40 % | **40 to 50 %** | **Moderate** excess water |
| **Adequate** | 16 to 20 % | **25 to 39 %** | **Ideal** moisture condition |
| Semi-dry | 10 to 15 % | **15 to 24 %** | **Moderate** water deficiency |
| Dry | < 10 % | **< 15 %** | **High** water deficiency |

### EXAMPLES

### Example 1 - Obtaining eucalyptus clonal seedlings

The process of the invention, applied to obtain eucalyptus clonal seedlings in a bioreactor by temporary immersion technique, comprises the following steps:
**Step 1** - Plant material remains at rest during the interval between immersion of the plant in culture media and gas exchange of the atmosphere of the internal compartment of the bioreactor. The medium is stored in the outer container (lower) and the explants in the inner compartment (above). The active photosynthetic radiation bioreactor is made of tubular fixtures with LED lamps in blue, red and white colors.
**Step 2** - The solenoid valve is activated by a timer in a range of pre-set time and duration. This allows entry of compressed air into the outer container, increasing pressure in the outer compartment (lower), causing the liquid culture medium to be transferred to the inner chamber (upper) of the plants, via the connection channels between the containers, and comes into contact with the base of the cultures. The management of temporary immersion varies with the type of eucalyptus clone and also with the stage of the culture. In the multiplication phase of shoots, 2-3 hours between temporary immersions and immersions

### Example 1 - Obtaining eucalyptus clonal seedlings

The process of the invention, applied to obtain eucalyptus clonal seedlings in a bioreactor by temporary immersion technique, comprises the following steps:
**Step 1** - Plant material remains at rest during the interval between immersion of the plant in culture media and gas exchange of the atmosphere of the internal compartment of the bioreactor. The medium is stored in the outer container (lower) and the explants in the inner compartment (above). The active photosynthetic radiation in the bioreactor is made of tubular fixtures with LED lamps in blue, red and white colors.
**Step 2** - The solenoid valve is activated by a timer in a range of pre-set time and duration. This allows entry of compressed air into the outer container, increasing pressure in the outer compartment (lower), causing the liquid culture medium to be transferred to the inner chamber (upper) of the plants, via the connection channels between the containers, and comes into contact with the base of the cultures. The management of temporary immersion varies with the type of eucalyptus clone and also with the stage of the culture. In the multiplication phase of shoots, 2-3 hours between temporary immersions and immersions in the red range (600-700 nm) and in the blue (400-500 nm), therefore, the management of illumination is controlled to provide a ratio of 3/1 (red/blue) to 2/1 (red/blue), respectively for multiplication and elongation. The intensity of PAR also varies according to the physiological stage of the culture. Typically, in the multiplication phase 20-30 µmol/m2/s is used, and in the elongation phase 40-60 µmol/m2/s is used.

## Claims

1. Process for in vitro micropropagation of plant material of a species of *eucalyptus* comprising the steps of multiplication and elongation of shoots developed from plant seedlings by the temporary immersion method, comprising:
(a) In the step of multiplication of the shoots, the material to be propagated is spread on a suitable support in a first container (vegetative propagation container) and is subjected to management of temporary immersion in a liquid nutrient medium from a second container (storage container of liquid medium), which is supplemented inside the plant containers with air injection and carbon dioxide enrichment and photosynthetically active radiation by way of light-emitting diode lamps, said management comprising alternated periods of immersion and rest, wherein said carbon dioxide enrichment is in the concentration of 800-1000 micromole CO₂ per mole of air and said photosynthetically active radiation is in the intensity of 20-30 umol/m²/s;
(b) In the step of shoots elongation, the propagation material is subjected to management of temporary immersion, preferably employing the same nutrient medium and the same conditions of operation in the bioreactor, according to step (a), wherein said photosynthetically active radiation is in the intensity of 40-60 µmol/m²/s, said management comprising of alternated periods of immersion and rest, wherein said method in said step (b) (elongation), said immersion periods are significantly shorter and said rest periods are longer compared with the periods of immersion and rest, respectively, in step (a) (of multiplication);
wherein said immersion period in step (a) is a maximum of 3 minutes and said rest period is at most 4 hours;
wherein said immersion period in step (b) is a maximum of 60 seconds and said rest period is at most 6 hours.

2. Process according to claim 1, wherein said immersion period is from 1 to 2 minutes and said rest period is from 2 to 3 hours.

3. Process according to any one of the preceding claims, wherein said immersion period is 20 to 30 seconds and said rest period is 4 to 5 hours.

4. Process according to any one of the preceding claims, wherein the in vitro micropropagation of the plant material is performed in a bioreactor of the BIT type.

5. Process for the production, on a large scale and high volume, of clonal plant seedlings ready for field development, said method comprises the steps of: (i) performing vegetative propagation of plant material by the process as defined in claims 1 to 4; (ii) allows the rooting and acclimatization of the elongated shoots obtained in step (i) in containers suitable for conditions to maintain plant turgor and reduce evaporative demand; and (iii) enable the growth and acclimatization of the microcuttings obtained in step (ii) until the size of the clonal seedlings is appropriate for transfer to the field.

6. Process according to claim 5, wherein the step (ii) is carried out in a humid environment, in which the water vapor droplets are so small as to remain suspended in the air until evaporated, while at the same time avoiding leaf wetness.

## Patentansprüche

1. Verfahren zur In-vitro-Mikrovermehrung von Pflanzenmaterial einer *Eukalyptusart,* umfassend die Schritte der Vermehrung und Verlängerung von Sprossen, die aus Pflanzensämlingen durch das temporäre Immersionsverfahren entwickelt wurden, umfassend:
(a) Im Schritt der Vermehrung der Sprossen wird das zu vermehrende Material auf einer geeigneten Unterlage in einem ersten Behälter (vegetativer Vermehrungsbehälter) ausgebreitet und einer Bewirtschaftung durch zeitweiliges Eintauchen in ein flüssiges Nährmedium aus einem zweiten Behälter (Vorratsbehälter für flüssiges Medium) unterzogen, das im Inneren der Pflanzenbehälter mit Lufteinblasung und Kohlendioxidanreicherung und photosynthetisch aktiver Strahlung mittels LED-Lampen ergänzt wird, wobei die Bewirtschaftung abwechselnde Perioden des Eintauchens und der Ruhe umfasst, wobei die Kohlendioxidanreicherung in der Konzentration von 800-1000 Mikromol CO₂ pro Mol Luft liegt und die photosynthetisch aktive Strahlung in der Intensität von 20-30 µmol/m²/s liegt;
(b) Im Schritt der Sprossverlängerung wird das Vermehrungsmaterial einem Management der zeitweiligen Immersion unterworfen, vorzugsweise unter Verwendung desselben Nährmediums und derselben Betriebsbedingungen im Bioreaktor, gemäß Schritt (a), wobei die photosynthetisch aktive Strahlung in der Intensität von 40-60 µmol/m²/s liegt, wobei das Verfahren im Schritt (b) (Verlängerung) die Immersionsperioden signifikant kürzer und die Ruheperioden länger sind, verglichen mit den Immersions- bzw. Ruheperioden im Schritt (a) (der Vermehrung);wobei die Eintauchzeit in Schritt (a) maximal 3 Minuten und die Ruhezeit maximal 4 Stunden beträgt; wobei die Eintauchzeit in Schritt (b) maximal 60 Sekunden und die Ruhezeit maximal 6 Stunden beträgt.

2. Verfahren nach Anspruch 1, wobei die Eintauchzeit 1 bis 2 Minuten und die Ruhezeit 2 bis 3 Stunden beträgt.

3. Verfahren nach einem der vorhergehenden Ansprüche, wobei die Eintauchzeit 20 bis 30 Sekunden und die Ruhezeit 4 bis 5 Stunden beträgt.

4. Verfahren nach einem der vorhergehenden Ansprüche, wobei die in vitro-Mikrovermehrung des Pflanzenmaterials in einem Bioreaktor vom BIT-Typ durchgeführt wird.

5. Verfahren zur Herstellung von klonalen Pflanzensetzlingen, die für die Feldentwicklung bereit sind, in großem Maßstab und in großen Mengen, wobei das Verfahren die folgenden Schritte umfasst: (i) Durchführen einer vegetativen Vermehrung von Pflanzenmaterial nach dem Verfahren gemäß den Ansprüchen 1 bis 4; (ii) Ermöglichen des Bewurzelns und der Akklimatisierung der in Schritt (i) erhaltenen länglichen Sprossen in Behältern, die für Bedingungen geeignet sind, die den Pflanzenturgor aufrechterhalten und den Verdunstungsbedarf verringern; und (iii) Ermöglichen des Wachstums und der Akklimatisierung der in Schritt (ii) erhaltenen Mikrostecklinge, bis die Größe der klonalen Sämlinge für den Transfer ins Feld geeignet ist.

6. Verfahren nach Anspruch 5, wobei der Schritt (ii) in einer feuchten Umgebung durchgeführt wird, in der die Wasserdampftröpfchen so klein sind, dass sie in der Luft suspendiert bleiben, bis sie verdampft sind, während gleichzeitig die Blattnässe vermieden wird.

## Revendications

1. Procédé de micropropagation in vitro de matériel végétal d'une espèce d'*eucalyptus* comprenant les étapes de multiplication et d'allongement des pousses développées à partir de plants végétaux par la méthode d'immersion temporaire, comprenant :
(a) Lors de l'étape de multiplication des pousses, le matériel à propager est étalé sur un support approprié dans un premier conteneur (conteneur de propagation végétative) et est soumis à la gestion d'une immersion temporaire dans un milieu nutritif liquide à partir d'un second conteneur (conteneur de stockage de milieu liquide), qui est complétée à l'intérieur des conteneurs de plantes par une injection d'air et un enrichissement en dioxyde de carbone et un rayonnement photosynthétique actif au moyen de lampes à diodes électroluminescentes, ladite gestion comprenant des périodes alternées d'immersion et de repos, dans laquelle ledit enrichissement en dioxyde de carbone est à la concentration de 800-1000 micromole de CO₂ par mole d'air et ledit rayonnement photosynthétique actif est à l'intensité de 20-30 µmol/m²/s;
(b) Dans l'étape d'allongement des pousses, le matériel de propagation est soumis à une gestion d'immersion temporaire, en utilisant de préférence le même milieu nutritif et les mêmes conditions de fonctionnement dans le bioréacteur, selon l'étape (a), dans laquelle ledit rayonnement photosynthétiquement actif est d'une intensité de 40-60 µmol/m²/s, ladite gestion comprenant des périodes alternées d'immersion et de repos, dans laquelle ledit procédé dans ladite étape (b) (d'allongement), lesdites périodes d'immersion sont sensiblement plus courtes et lesdites périodes de repos sont plus longues par rapport aux périodes d'immersion et de repos, respectivement, dans l'étape (a) (de multiplication) ;dans laquelle ladite période d'immersion à l'étape (a) est de 3 minutes au maximum et ladite période de repos est de 4 heures au maximum; dans laquelle ladite période d'immersion de l'étape (b) est de 60 secondes au maximum et ladite période de repos est de 6 heures au maximum.

2. Procédé selon la revendication 1, dans lequel ladite période d'immersion est de 1 à 2 minutes et ladite période de repos est de 2 à 3 heures.

3. Procédé selon l'une des revendications précédentes, dans lequel ladite période d'immersion est de 20 à 30 secondes et ladite période de repos de 4 à 5 heures.

4. Procédé selon l'une quelconque des revendications précédentes, dans lequel la micropropagation in vitro du matériel végétal est effectuée dans un bioréacteur de type BIT.

5. Procédé pour la production, à grande échelle et en grand volume, de plants de plantes clonales prêts à être mis en terre, ladite méthode comprend les étapes suivantes (i) effectuer la multiplication végétative du matériel végétal par le procédé défini dans les revendications 1 à 4 ; (ii) permettre l'enracinement et l'acclimatation des pousses allongées obtenues dans l'étape (i) dans des conteneurs adaptés aux conditions de maintien de la turgescence de la plante et de réduction de la demande d'évaporation ; et (iii) permettre la croissance et l'acclimatation des microboutures obtenues dans l'étape (ii) jusqu'à ce que la taille des plantules clonales soit appropriée pour le transfert au champ.

6. Procédé selon la revendication 5, dans lequel l'étape (ii) est réalisée dans un environnement humide, dans lequel les gouttelettes de vapeur d'eau sont si petites qu'elles restent en suspension dans l'air jusqu'à évaporation, tout en évitant l'humidité des feuilles.
